(19) Europäisches Patentamt

European Patent Office

Office européen des brevets

(11) Veröffentlichungsnummer: **0 418 647 B1**

## (12) EUROPÄISCHE PATENTSCHRIFT

(45) Veröffentlichungstag der Patentschrift: **14.12.94**

(51) Int. Cl.5: **C07K 3/28**

(21) Anmeldenummer: **90117038.1**

(22) Anmeldetag: **05.09.90**

---

(54) **Verfahren zur Reinigung von Plasminogen-Aktivator-Inhibitor 2 (PAI-2).**

---

(30) Priorität: **06.09.89 DE 3929504**

(43) Veröffentlichungstag der Anmeldung:
**27.03.91 Patentblatt 91/13**

(45) Bekanntmachung des Hinweises auf die
Patenterteilung:
**14.12.94 Patentblatt 94/50**

(84) Benannte Vertragsstaaten:
**AT BE CH DE DK ES FR GB GR IT LI LU NL SE**

(56) Entgegenhaltungen:
**DE-A- 3 713 272**

**BIOL. CHEM. HOPPE-SEYLER, Band 368, Oktober 1987; T.W. STIEF et al., Seiten 1427-1433&NUM;**

(73) Patentinhaber: **BEHRINGWERKE Aktiengesellschaft**
**Postfach 1140**
**D-35001 Marburg (DE)**

(72) Erfinder: **Radtke, Klaus-Peter, Dr.**
**10666 North Torrey Pines Road**
**La Jolla, CA 92037 (US)**
Erfinder: **Heimburger, Norbert, Prof. Dr.**
**Sonnenhang 10**
**D-3550 Marburg (US)**
Erfinder: **Wenz, Karlheinz**
**Talstrasse 10**
**D-3556 Weimar (DE)**

---

**Beschreibung**

Die Erfindung betrifft ein Verfahren zur Reinigung von Plasminogen-Aktivator-Inhibitor 2 (PAI-2), wobei Verunreinigungen mit einer Acridin- oder Chinolinbase ausgefällt werden.

Plasminogen-Aktivatoren (PA) sind Serinproteasen, die das fibrinolytische System aktivieren. Sie wandeln das inaktive Proenzym Plasminogen in das aktive Enzym Plasmin um, das Fibrin und Fibrinogen abbaut. Im Organismus gibt es zwei PA, den Gewebeaktivator (tissue plasminogen activator, t-PA) und der mit Urokinase identische PA (u-PA), der von Nierenzellen, aber auch von anderen normalen oder malignen Zellen produziert wird. Dem t-PA kommt eine besondere Bedeutung bei der Verhütung von Thrombosen zu. u-PA verhindert die Bildung von Fibringerinnseln in den ableitenden Harnwegen, trägt aber auch zur Wundheilung bei und ist bei neoplastischem Wachstum gefunden worden.

Konzentration und Aktivität der PA werden durch ihre Synthese, aber auch durch Inhibitoren reguliert. Zwei spezifische PA-Inhibitoren (PAI) sind bekannt, die Plasmin oder andere Proteasen nicht inhibieren. Einer wird in den Endothelzellen produziert (PAI-1), der andere in Plazenta (PAI-2) wurde auch "Minactivin" genannt.

Ausgehend von einem Extrakt aus Placenta kann nach dem Stand der Technik durch eine Ammonsulfat-Fraktionierung, Adsorption von Verunreinigungen an CM-$^R$Sephadex C-50, eine Gelfiltration und Hydroxylapatit-Chromatographie, eine 120fache Anreicherung von PAI-2 erreicht werden. Dabei fallen zwei Formen von PAI-2 an mit Molekulargewichten von 70 und 43 kDa. Es wurde auch die Immunaffinitätschromatographie sowie eine Kombination von Immunaffinitäts- und FPLC-Chromatographie zur Reinigung benutzt. Eine Kombination von 8 Verfahrensschritten, die eine Fällung mit Neutralsalzen, Chromatographien an CM- und DEAE-$^R$Sepharose und Hydroxylapatit sowie eine präparative Elektrophorese enthält, führte ausgehend von Placenta, zu einem reinen PAI-2 mit einem Molekulargewicht von 47 kDa. Es war auch bekannt, daß chromatographische Reinigungsschritte durch den Zusatz von Reduktionsmitteln vom Typ des Dithiothreitols vorteilhaft beeinflußt werden können.

Aufgabe der Erfindung ist das Auffinden eines einfacheren, im Produktionsmaßstab brauchbaren Verfahrens für die Gewinnung von PAI-2.

Überraschenderweise wurde gefunden, daß mit 2-Ethoxy-6,9-diaminoacridin-lactat ($^R$Rivanol) aus einer Lösung von PAI-2 Verunreinigungen ausgefällt werden können, während der PAI-2 selbst im Überstand gelöst bleibt.

Als vorteilhaft erwies sich eine Vorinkubation mit Dithiothreitol (DTT).

Gegenstand der Erfindung ist ein Verfahren zur Reinigung von Plasminogen-Aktivator-Inhibitor 2 (PAI-2), dadurch gekennzeichnet, daß eine PAI-2-haltige Lösung mit einem Disulfidbrücken spaltenden Agenz, vorzugsweise Dithiothreitol (DTT) vorinkubiert und mit soviel einer geeigneten wasserlöslichen Acridin- oder Chinolinbase, vorzugsweise 2-Ethoxy-6,9-diaminoacridin-lactat versetzt, daß der gebildete Niederschlag höchstens wenig PAI-2 enthält, dieser Niederschlag abgetrennt und der PAI-2 aus dem Überstand gewonnen und gegebenenfalls mit bekannten Verfahren weiter gereinigt wird.

Bei Zugabe niederer Mengen an Acridin- oder Chinolinbase bezogen auf die gelöste Menge an Proteinen wird auch PAI-2 ausgefällt. Zweckmäßig ist eine Konzentration an einem wasserlöslichen Salz der Base, vorzugsweise 2-Ethoxy-6,9-diaminoacridin-lactat von 200 mg bis 2 g/g Protein in der Lösung (PAI-2 bleibt weitgehend in Lösung). Es wird bei einem pH von 5,5 - 8,5 gearbeitet.

Die PAI-2-haltige Lösung kann vorher gegebenenfalls unter Zugabe von Stabilisatoren, vorzugsweise Glycin und/oder Saccharose pasteurisiert worden sein.

Vorzugsweise ist die PAI-2-haltige Lösung ein Placentaextrakt oder eine Lösung enthaltend gentechnisch hergestellten PAI-2.

Das Disulfide spaltende Agenz wird in einer Konzentration von 10 mmol/l bis 250 mmol/l, vorzugsweise 100 mmol/l verwendet. Es wird von 15 min. bis 3 Stunden, vorzugsweise 1 Stunde, bei 10 bis 40°C, vorzugsweise etwa 37°C vorinkubiert.

85 - 90 % der eingesetzten PAI-2-Aktivität, aber nur 5 - 8 % der eingesetzten Proteinmenge finden sich im Überstand, wenn wie beschrieben gearbeitet wird.

Aus dem Überstand nach der Fällung kann die Acridinbase, vorzugsweise mit NaCl, vorzugsweise 5 %, ausgesalzt und der PAI-2 durch eine Ammonsulfat-Fällung, beispielsweise durch 80 %ige Sättigung der Lösung mit Ammonsulfat konzentriert werden, die in Verbindung mit einer Vorfällung bei 30 % Sättigung der Lösung mit Ammonsulfat eine zusätzliche Reinigung bringt. Da durch die Rivanol- und Ammonsulfat-Fällung ca. 90 % des eingesetzten Proteins abgetrennt werden, kann die kleine Restmenge dann Reinigungsverfahren unterworfen werden, denen vom Volumen her Grenzen gesetzt sind: beispielsweise eine Chromatographie an DEAE-$^R$Affigel-Blue, ein Chromatographie-Gel mit bifunktionaler Affinität, das aus Diethylaminoethyl-Gruppen und $^R$Cibacron Blue F3GA-Farbstoff kovalent gebunden an Agarose besteht und an Hydroxylapatit. Ein Material dieser Reinheitsstufe dürfte bereits für einen therapeutischen Einsatz geeignet sein. Die Hochreinigung kann durch hydrophobe Chromatographie an einer Phenylalanin-Säule vorgenommen werden, ist aber mit beträchtlichen Verlusten verbunden.

Der gewonnene PAI-2 kann gegebenenfalls zusätzlich pasteurisiert werden, falls nicht vorher geschehen.

Nachfolgend wird das erfindungsgemäße Verfahren erläutert:

Für die Überwachung der Gewinnung von PAI-2 wurde eine amidolytische Methode in Verbindung mit dem chromogenen Substrat der Firma Kabi S-2444 (Glu-Gly-Arg-pNA) verwendet.

Für die Bestimmung wurden 50 $\mu$l Urokinase (u-PA) (1000 E/ml) mit 100 $\mu$l PAI-2-haltiger Probe 4 min bei Raumtemperatur inkubiert und von dieser Mischung 80 $\mu$l in eine auf 37°C vorgewärmte Plastik-Küvette übertragen. Anschließend wurden 50 $\mu$l Puffer A und 20 $\mu$l S-2444 (6 mM) dazugegeben. Es wurde die Zunahme der Absorption bei 405 nm im [R]Cobas Bio Zentrifugal-Analyser bestimmt. Die Meßwerte wurden über den Vergleich mit einer Verdünnungskurve ausgewertet, die über eine Reihenverdünnung eines PAI-2-Hausstandards erstellt worden war.

Materialien, die für die Isolierung von PAI-2 verwendet wurden:

- 2-Ethoxy-6,9-diaminoacridin-lactat (6,9-Diamino-2-ethoxyacridin-lactat): Sigma Chemie GmbH, D-6100 Darmstadt; für die fraktionierte Fällung wurde eine 2,5 %ige (w/v) Lösung in 12,5 mM Tris, pH 6,8, verwendet.
- Dithiothreitol (DTT): Serva Feinbiochemika GmbH & Co., D-6900 Heidelberg
- Hydroxylapatit: [R]BioRad, Richmond, CA, USA
- Phenylalanin-[R]Sepharose und CNBr-aktivierte [R]Sepharose 4B: Deutsche Pharmacia GmbH, D-6900 Freiburg
- S-2444: Kabi Vitrum, S-11287 Stockholm, Schweden
- Tris(hydroxymethyl)amminomethan (Tris): E. Merck, D-6100 Darmstadt
- Urokinase ([R]Actosolv): Behringwerke AG, D-3550 Marburg
- Puffer A: 50 mM Tris, pH 8,4, 1 % Polygelin, 100 mM NaCl, 0,01 % [R]Triton X 100 und 0,01 % $NaN_3$
- Puffer B: 20 mM Tris, pH 7,5, 20 mM DTT
- Puffer C: 20 mM $Na_2HPO_4$, pH 6,8, 20 mM DTT
- Puffer D: 20 mM Tris, pH 7,5, 20 mM DTT, 30 % Ammonsulfat-Sättigung
- Puffer E: 20 mM Tris, pH 7,5, 100 mM NaCl
- Puffer F: 20 mM Tris, pH 6,8

Beispiel

Als Rohstoff für die Herstellung von PAI-2 wurde blutfrei gewaschene, gefrorene menschliche Plazenta verwendet, die in einem Cutter zerhackt und anschließend mit 0,5 % NaCl und 3 mM EDTA extrahiert worden war. Die Zelltrümmer wurden durch Zentrifugation entfernt und der Überstand mit 8 % Rivanol gefällt, das Präzipitat gelöst und einer fraktionierten Fällung mit Ammoniumsulfat unterworfen. Die Fällung, die den PAI-2 enthielt, wurde in Puffer F gelöst und dagegen dialysiert. Der konzentrierte Plazentaextrakt enthielt 4.615 E PAI-2/ml mit einer spezifischen Aktivität von 120 E/mg. Dieses Material wurde für die Hochreinigung eingesetzt.

- Rivanol- und Ammoniumsulfat-Fällung

500 ml des oben beschriebenen Ausgangsmaterials (Tab. 1) wurden mit 7,7 g DTT (Endkonzentration 100 mM) bei 37°C 1 Std. inkubiert. Dieser Lösung wurden tropfenweise und bei kontinuierlich vorsichtigem Rühren 1.520 ml einer 2,5 %igen Rivanollösung zugesetzt und damit eine Endkonzentration entsprechend 200 % erreicht. Das aus der Fällung resultierende Präzipitat wurde durch Zentrifugation entfernt und überschüssiges Rivanol aus dem Überstand durch Zugabe von 100 g festem NaCl auf eine Endkonzentration von 5 % abgetrennt. Dann wurde zu dem Überstand 343 g festes Ammoniumsulfat zugegeben, bis eine Endkonzentration entsprechend einer 30 %igen Sättigung erreicht wurde. Die Fällung wurde durch Zentrifugation (20 min, 3000 U/min) abgetrennt und anschließend 694 g festes Ammoniumsulfat bis auf eine Endkonzentration einer 80 %igen Sättigung zugesetzt. Der dabei anfallende Niederschlag wurde im Puffer B konzentriert gelöst und dialysiert. Daraus resultierten 80 ml gelöster und dialysierter Ammoniumsulfat-Rückstand.

- DEAE-[R]Affigel-Blue-Chromatographie

80 ml des Ammoniumsulfat-Präzipitats wurden auf eine DEAE-Affigel-Blue-Säule (17,5 x 4,4 cm, 260 ml Gelbett), die mit Puffer B äquilibriert worden war, aufgetragen. Die Säule wurde mit einem NaCl-Gradienten in Puffer B (2 x 1000 ml), der sich über einen Bereich von 0 bis 200 mM NaCl erstreckte, bei einer Flußgeschwindigkeit von 90 ml/Std. eluiert. Die Eiweißkonzentration wurde fortlaufend über die OD bei 280 nm gemessen und die PAI-2-Aktivität wie beschrieben bestimmt.

- Hydroxylapatit-Chromatographie

472 ml der PAI-2-enthaltenden Fraktionen aus dem vorhergehenden Schritt wurden gesammelt, dialysiert gegen Puffer C und auf eine Hydroxylapatit-Säule (15 x 3,2 cm, 56 ml Gelbett) aufgetragen (Tab. 1), die mit dem gleichen Puffer äquilibriert worden war. Die Säule wurde mit einer Flußgeschwindigkeit von 50 ml/Std. und mit einem Salzgradienten aus Natrium-Phosphat (0,02 bis 0,3 M; 2 x 250 ml) in Puffer C eluiert. Die Einweißkonzentration und PAI-2-Aktivität des Eluats wurden kontinuierlich bestimmt.

- Phenylalanin-[R]Sepharose

Die PAI-2-haltigen Fraktionen nach Hydroxylapatit-Chromatographie wurden gesammelt und zu 120 ml dieses Materials 13,2 g festes Ammoniumsulfat zugesetzt, um eine 30 %ige Sättigung zu erreichen. Diese Lösung wurde auf eine Phenylalanin-[R]Sepharose-Säule aufgetragen, die mit dem Puffer D äquilibriert worden war. Das Harz wurde mit einer Flußgeschwindigkeit von 20 ml/Std. und einem Gradienten, gebildet aus Puffer D und B (2 x 100 ml), eluiert. Die PAI-2-haltigen Fraktionen wurden gesammelt und charakterisiert. Das reinste Material besaß eine spezifische Aktivität von 60.000 E(=Einheiten)/mg und wanderte in der SDS-Polyacrylamid-Gelelektrophorese als eine Bande mit einem Molekulargewicht von 43 kDa, die sich im Immunoblot mit einem spezifischen monoklonalen Antikörper darstellen ließ.

Tabelle 1

Reinigung von Plasminogen-Aktivator-Inhibitor 2 (PAI-2)

| | Volumen (ml) | Protein-konz. (mg) | PAI-2-Aktivität (E) | Spez.Aktivität (E/mg) | Reinigungs-faktor | Ausbeute (%) |
|---|---|---|---|---|---|---|
| Ausgangs-material | 500 | 19.000 | $2,20 \times 10^6$ | 115 | 1 | 100 |
| Rivanol-Überstand | 1.850 | 1.311 | $1,80 \times 10^6$ | 1.373 | 11,9 | 82 |
| 30-80% Ammoniumsulfat-Rückstand | 80 | 800 | $1,15 \times 10^6$ | 1.460 | 13 | 52 |
| DEAE-$^R$Affi-gel-Blue | 472 | 65 | $5,90 \times 10^5$ | 9.143 | 79,5 | 27 |
| Hydroxylapatit | 120 | 16,3 | $4,60 \times 10^5$ | 28.100 | 244 | 21 |
| Phenylalanin-$^R$Sepharose | 210 | 2,3 | $1,20 \times 10^4$ | 52.300 | 455 | 5,5 |

## Patentansprüche

1. Verfahren zur Reinigung von Plasminogen-Aktivator-Inhibitor 2 (PAI-2), dadurch gekennzeichnet, daß eine PAI-2-haltige Lösung bei einem pH-Wert von 5,5 - 8,5 und einer Temperatur von 10 bis 40 °C mit einem Disulfidbrücken spaltenden Agenz in einer Konzentration von 10 mmol/l bis 250 mmol/l 15 min. bis 3 Stunden vorinkubiert und mit soviel einer geeigneten wasserlöslichen Acridin- oder Chinolinbase, vorzugsweise 2-Ethoxy-6,9-diaminoacridin-lactat versetzt wird, daß ein Niederschlag gebildet wird, wobei mehr als 85 % der eingesetzten PAI-2-Aktivität im Überstand bleiben, dieser Niederschlag abgetrennt und der PAI-2 aus dem Überstand gewonnen und gegebenenfalls mit bekannten Verfahren weiter gereinigt wird.

2. Verfahren nach Anspruch 1, dadurch gekennzeichnet, daß die PAI-2-haltige Lösung ein Placentaextrakt oder eine Lösung von gentechnisch hergestelltem PAI-2 ist.

3. Verfahren nach Anspruch 1, dadurch gekennzeichnet, daß das Disulfide spaltende Agenz Dithiothreitol ist.

4. Verfahren nach Anspruch 1, dadurch gekennzeichnet, daß das Disulfide spaltende Agenz in einer Konzentration von 100 mmol/l verwendet wird.

5. Verfahren nach Anspruch 1, dadurch gekennzeichnet, daß 1 Stunde vorinkubiert wird.

6. Verfahren nach Anspruch 1, dadurch gekennzeichnet, daß bei etwa 37°C vorinkubiert wird.

7. Verfahren nach Anspruch 1, dadurch gekennzeichnet, daß die Base in gelöster Form zugesetzt wird.

8. Verfahren nach Anspruch 1, dadurch gekennzeichnet, daß eine PAI-2-haltige Lösung pasteurisiert wird.

## Claims

1. A process for the purification of plasminogen activator inhibitor 2 (PAI-2), which comprises a PAI-2-containing solution being preincubated for 15 min to 3 hours at a pH of 5.5-8.5 and a temperature of 10 to 40°C with an agent cleaving disulfide linkages in a concentration of from 10 mmol/l to 250 mmol/l, and being mixed with a suitable water-soluble acridine or quinoline base, preferably 2-ethoxy-6,9-diaminoacridine lactate, in such an amount that a precipitate is formed, with more than 85% of the PAI-2 activity employed remaining in the supernatant, this precipitate being separated off and the PAI-2 being obtained from the supernatant and, where appropriate, further purified using known processes.

2. The process as claimed in claim 1, wherein the PAI-2-containing solution is an extract of placenta or a solution of genetically engineered PAI-2.

3. The process as claimed in claim 1, wherein the disulfide-cleaving agent is dithiothreitol.

4. The process as claimed in claim 1, wherein the disulfide-cleaving agent is used in a concentration of 100 mmol/l.

5. The process as claimed in claim 1, wherein preincubation is carried out for 1 hour.

6. The process as claimed in claim 1, wherein preincubation is carried out at about 37°C.

7. The process as claimed in claim 1, wherein the base is added in dissolved form.

8. The process as claimed in claim 1, wherein a PAI-2-containing solution is pasteurized.

## Revendications

1. Procédé de purification de l'inhibiteur 2 de l'activateur du plasminogène (PAI-2), caractérisé en ce qu'on fait préincuber, à pH 5,5 - 8,5 et à une température de 10 à 40°C pendant 15 min à 3 h, une solution contenant du PAI-2 avec un agent coupant les ponts disulfure, à une concentration de 10 mmoles/l à 250 mmoles/l, et on mélange le tout avec autant qu'il le faut d'une base d'acridine ou de quinoléine hydrosoluble appropriée, de préférence le lactate de 2-éthoxy-6,9-diaminoacridine, pour qu'un précipité se forme, plus de 85 % de l'activité de PAI-2 introduit restant dans le surnageant, on sépare ce précipité et on récupère à partir du surnageant le PAI-2, dont on poursuit éventuellement la purification au moyen de procédés connus.

2. Procédé selon la revendication 1, caractérisé en ce que la solution contenant PAI-2 est un extrait placentaire ou une solution de PAI-2 fabriquée par génie génétique.

3. Procédé selon la revendication 1, caractérisé en ce que l'agent coupant le disulfure est le dithiothréitol.

4. Procédé selon la revendication 1, caractérisé en ce que l'agent coupant le disulfure est utilisé à une concentration de 100 mmoles/l.

5. Procédé selon la revendication 1, caractérisé en ce qu'on procède à une préincubation de 1 heure.

6. Procédé selon la revendication 1, caractérisé en ce que la préincubation est effectuée à environ 37°C.

7. Procédé selon la revendication 1, caractérisé en ce la base est ajoutée sous forme dissoute.

8. Procédé selon la revendication PAI, caractérisé en ce qu'on pasteurise une solution contenant PAI-2.